# DEMANDE DE BREVET EUROPEEN

(11) **EP 4 191 675 A1**
(43) Date de publication de la demande: **07.06.2023**
(21) Numéro de dépôt: 22208821.3
(22) Date de dépôt: 22.11.2022
(51) Int. Cl.: H01L 27/146, G01T 1/20

(54) **DISPOSITIF D'IMAGERIE A RAYONS X**

(30) Priorité: 02.12.2021 FR 2112877
(71) Demandeur: Commissariat à l'énergie atomique et aux énergies alternatives, 75015 Paris (FR)
(72) Inventeur: TEMPLIER, François, 38054 GRENOBLE CEDEX 09 (FR); VERGER, Loick, 38054 GRENOBLE CEDEX 09 (FR); GROS-DAILLON, Eric, 38054 GRENOBLE CEDEX 09 (FR); BECKER, Sébastien, 38054 GRENOBLE CEDEX 09 (FR)
(74) Mandataire: Cabinet Beaumont

(57) **Abrégé**

La présente description concerne un dispositif d'imagerie à rayons X, comportant :
- un substrat de report (100) comportant des éléments de connexion électrique ;
- une matrice de pixels comportant chacun une puce élémentaire monolithique (153) fixée et connectée électriquement à des éléments de connexion électrique du substrat de report (100), et une photodiode (PD) formée sur le substrat de report et connectée électriquement à la puce élémentaire (153) ; et
- un scintillateur (180) revêtant la matrice de pixels,
dans lequel, dans chaque pixel, la puce élémentaire (153) comprend un circuit intégré de lecture de la photodiode (PD) du pixel.

## Description

### Domaine technique

La présente description concerne un dispositif d'imagerie à rayons X et un procédé de fabrication d'un tel dispositif, notamment pour des applications de radiographie, par exemple dans le domaine de l'imagerie médicale.

### Technique antérieure

Parmi les dispositifs d'imagerie à rayons X connus, on peut distinguer les dispositifs à conversion indirecte et les dispositifs à conversion directe.

Les dispositifs à conversion indirecte comprennent une matrice de photodiodes adaptées à capter un rayonnement lumineux, et un scintillateur disposé au-dessus de la matrice de photodiodes. En fonctionnement, le scintillateur émet de la lumière suite à l'absorption des rayons X. La lumière émise par le scintillateur est convertie en charges électriques par les photodiodes. Ainsi, la matrice de photodiodes acquiert une image représentative de la distribution lumineuse émise par le scintillateur, cette distribution lumineuse étant elle-même représentative de la distribution de rayon X reçue par le scintillateur.

Les dispositifs à conversion directe comprennent une couche d'un matériau semiconducteur de conversion adapté à convertir directement les rayons X absorbés, en charges électrique. La couche de conversion est disposée au-dessus d'une matrice de circuits élémentaires adaptée à lire les charges électriques générées dans le matériau de conversion. En fonctionnement la couche de conversion génère des charges électriques suite à l'absorption des rayons X. Ces charges sont lues par la matrice de circuits de lecture. Ainsi, la matrice de circuits de lecture acquiert directement une image représentative de la distribution de rayons X reçue par le matériau de conversion.

On s'intéresse ici plus particulièrement à la réalisation de dispositifs d'imagerie à rayons X à conversion indirecte.

### Résumé de l'invention

Un mode de réalisation prévoit un dispositif d'imagerie à rayons X, comportant :
- un substrat de report comportant des éléments de connexion électrique ;
- une matrice de pixels comportant chacun une puce élémentaire monolithique fixée et connectée électriquement à des éléments de connexion électrique du substrat de report, et une photodiode formée sur le substrat de report et connectée électriquement à la puce élémentaire ; et
- un scintillateur revêtant chaque pixel,
dans lequel, dans chaque pixel, la puce élémentaire comprend un circuit intégré de lecture de la photodiode du pixel.

Selon un mode de réalisation, dans chaque puce élémentaire, le circuit intégré de lecture de la photodiode du pixel est réalisé en technologie CMOS.

Selon un mode de réalisation, dans chaque pixel, la photodiode comprend un empilement actif à base d'un matériau semiconducteur inorganique, par exemple du silicium amorphe ou de l'oxyde d'indium-gallium-zinc.

Selon un mode de réalisation, dans chaque pixel, la photodiode comprend un empilement actif de diode photosensible organique.

Selon un mode de réalisation, dans chaque pixel, la photodiode comprend une électrode supérieure en un matériau transparent.

Selon un mode de réalisation, dans chaque pixel, la photodiode ne recouvre pas la puce élémentaire du pixel.

Selon un mode de réalisation, dans chaque pixel, la photodiode recouvre la puce élémentaire du pixel.

Selon un mode de réalisation, dans chaque pixel, la puce élémentaire du pixel comprend une LED inorganique et un circuit intégré de contrôle de la LED.

Un autre mode de réalisation prévoir un assemblage comportant des premier et deuxième dispositifs d'imagerie à rayons X tels que définis ci-dessus, superposés.

Selon un mode de réalisation, l'assemblage comprend une couche de filtrage entre les premier et deuxième dispositifs.

Un autre mode de réalisation prévoit un procédé de fabrication d'un dispositif d'imagerie à rayons X tel que défini ci-dessus, dans lequel les puces élémentaires sont reportées et fixées collectivement au substrat de report, au moyen d'un substrat de support temporaire.

Selon un mode de réalisation, le procédé comprend la formation des photodiodes des pixels sur le substrat de report avant l'étape de report collectif des puces élémentaires sur le substrat de report.

Selon un mode de réalisation, le procédé comprend la formation des photodiodes des pixels sur le substrat de report après l'étape de report collectif des puces élémentaires sur le substrat de report.

### Brève description des dessins

Ces caractéristiques et leurs avantages, ainsi que d'autres, seront exposés en détail dans la description suivante de modes de réalisation particuliers faite à titre non limitatif en relation avec les figures jointes parmi lesquelles :
les figures 1A, 1B, 1C, 1D, 1E, 1F, 1G, 1H et 1I sont des vues de dessus et en coupe illustrant des étapes d'un exemple d'un procédé de fabrication d'un dispositif d'imagerie à rayons X selon un mode de réalisation ;
les figures 2A, 2B, 2C, 2D et 2E sont des vues en coupe illustrant des étapes d'un exemple d'un procédé de fabrication de puces élémentaires de pixel d'un dispositif d'imagerie à rayons X selon un mode de réalisation ;
les figures 3A, 3B, 3C, 3D, 3E et 3F sont des vues de dessus et en coupe illustrant des étapes d'un autre exemple d'un procédé de fabrication d'un dispositif d'imagerie à rayons X selon un mode de réalisation ;
la figure 4 est une vue en coupe illustrant une variante du procédé des figures 3A, 3B, 3C, 3D, 3E et 3F ;
les figures 5A et 5B sont des vues de dessus et en coupe illustrant des étapes d'un autre exemple d'un procédé de fabrication d'un dispositif d'imagerie à rayons X selon un mode de réalisation ;
les figures 6A et 6B sont des vues de dessus et en coupe illustrant des étapes d'un autre exemple d'un procédé de fabrication d'un dispositif d'imagerie à rayons X selon un mode de réalisation ; et
la figure 7 est une vue en coupe illustrant une variante d'un dispositif d'imagerie à rayons X selon un mode de réalisation.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, les diverses applications que peuvent avoir les dispositifs d'imagerie à rayons X décrits n'ont pas été détaillées, les modes de réalisation décrits étant compatibles avec toutes ou la plupart des applications connues d'imagerie à rayons X, et tout particulièrement les applications pouvant tirer profit de dispositif d'imagerie à rayons X de grandes dimensions, par exemple des dispositifs de dimensions latérales supérieures à 10 cm et de préférence supérieures à 20 cm. En outre, la réalisation des diodes photosensibles, des circuits électroniques de contrôle, et du scintillateur des dispositifs décrits n'ont pas été détaillés, la réalisation de ces éléments étant à la portée de la personne du métier à partir des indications de la présente description. Par rayons X on entend ici, par exemple, des rayonnements constitués de photons d'énergie comprise, par exemple, entre 1000 eV (électron-volt) et 20 MeV (méga-électron-volt).

Sauf précision contraire, lorsque l'on fait référence à deux éléments connectés entre eux, cela signifie directement connectés sans éléments intermédiaires autres que des conducteurs, et lorsque l'on fait référence à deux éléments reliés (en anglais "coupled") entre eux, cela signifie que ces deux éléments peuvent être connectés ou être reliés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", etc., il est fait référence sauf précision contraire à l'orientation des vues en coupe des figures.

Sauf précision contraire, les expressions "environ", "approximativement", "sensiblement", et "de l'ordre de" signifient à 10 % près, de préférence à 5 % près.

Selon un aspect des modes de réalisation décrits, on prévoit un dispositif d'imagerie à rayons X comportant un substrat de report, une matrice de pixels de photo-détection formée sur le substrat de report, et un scintillateur revêtant la matrice de pixels de photo-détection. Chaque pixel de photo-détection comprend une photodiode formée sur le substrat de report et reliée ou connectée électriquement à des éléments de connexion électrique (piste, plages, bornes ou plots de connexion électrique) du substrat de report, et une puce élémentaire monolithique, fixée et connectée électriquement à des éléments de connexion électrique du substrat de report. Dans chaque pixel, la puce élémentaire est connectée à la photodiode, par exemple par au moins un élément de connexion électrique du substrat de report. La puce élémentaire comprend au moins un circuit intégré de lecture de la photodiode du pixel, de préférence réalisé en technologie CMOS.

Chaque puce élémentaire comporte une face de connexion comprenant une pluralité de plots (aussi appelées bornes ou plages) de connexion électrique destinés à être connectés au substrat de report pour la commande de la puce. Le substrat de report comprend une face de connexion comportant, pour chaque puce élémentaire, une pluralité de plots (aussi appelées bornes ou plages) de connexion électrique destinés à être connectés respectivement aux plots de connexion électrique de la puce. Les puces sont rapportées sur le substrat de report, faces de connexion tournées vers la face de connexion du substrat de report, et fixées sur le substrat de report de façon à connecter les plots de connexion électrique de chaque puce aux plots de connexion électrique correspondants du substrat de report.

Un avantage des modes de réalisation décrits est qu'ils permettent d'obtenir des dispositifs d'imagerie de grandes dimensions, par exemple de dimensions latérales supérieures à 10 cm et de préférence supérieures à 20 cm, à des coûts relativement faibles, tout en bénéficiant des avantages des circuits intégrés monolithiques, par exemple des circuits CMOS, pour la lecture des photodiodes. Un avantage réside notamment dans le faible bruit de lecture introduit par de tels circuits intégrés monolithiques par rapport à des circuits à base de transistors TFT (de l'anglais "Thin Film Transistor" - transistor en couches minces), formés par dépôts successifs de plusieurs couches minces directement sur le substrat de report. Un autre avantage est un gain en rapidité de lecture, lié à la meilleure mobilité des porteurs de charge dans de tels circuits intégrés monolithiques par rapport à des circuits TFT. En outre, de tels circuits permettent, de façon optionnelle, de mettre en œuvre des fonctions additionnelles de traitement des signaux électriques fournis par les photodiodes. Un autre avantage réside dans le faible encombrement des puces élémentaires monolithique par rapport à des circuits TFT.

Des exemples de réalisation d'un tel dispositif d'imagerie à rayons X vont être décrits plus en détail ci-après en relation avec les figures.

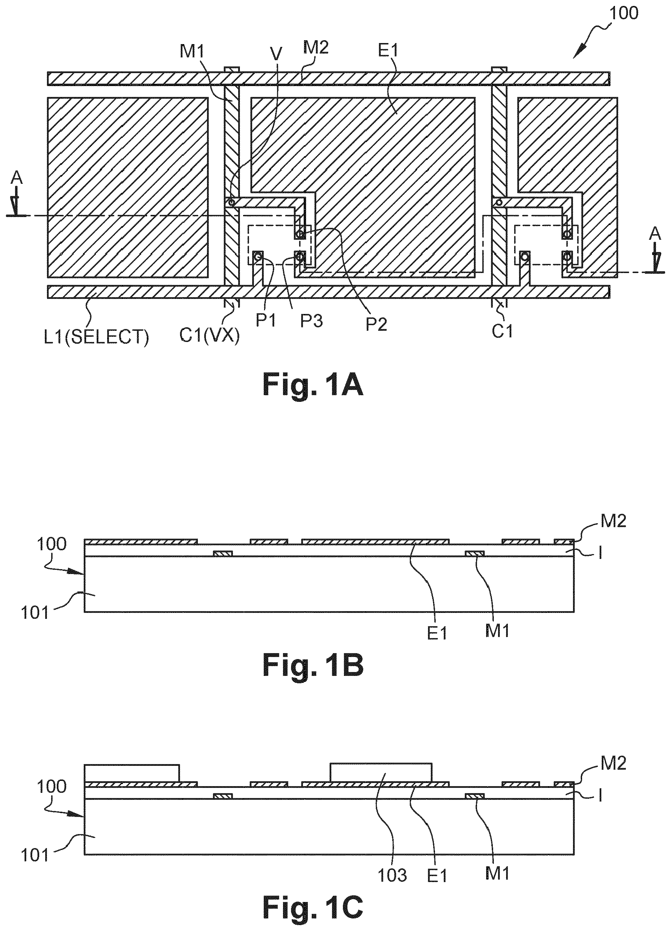

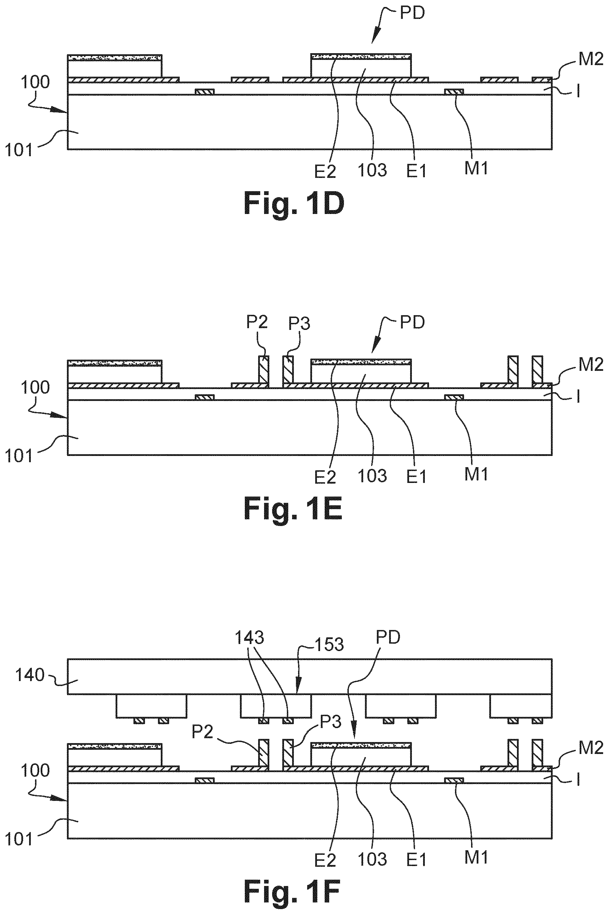

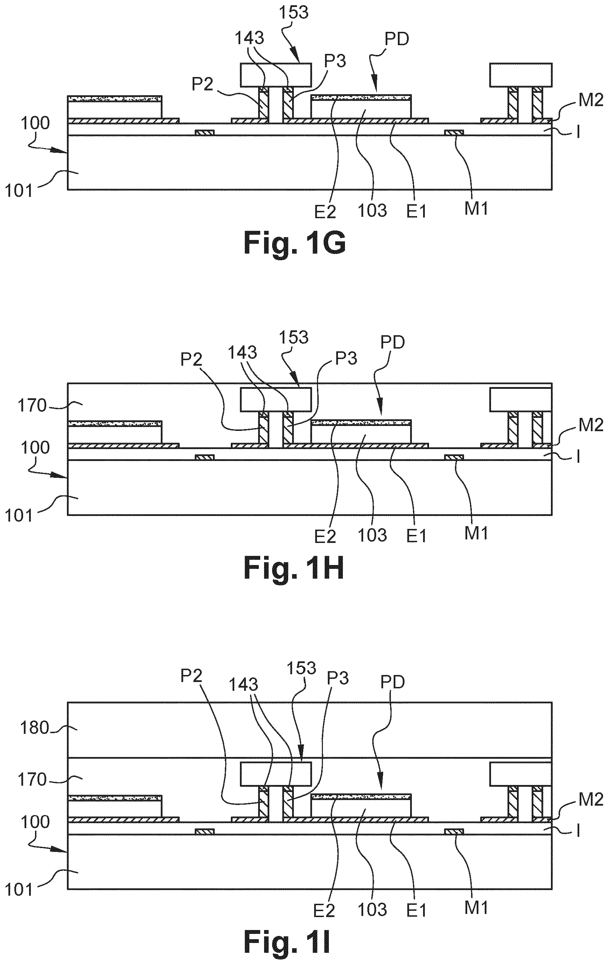

Les figures 1A, 1B, 1C, 1D, 1E, 1F, 1G, 1H et 1I sont des vues de dessus et en coupe illustrant des étapes d'un exemple d'un procédé de fabrication d'un dispositif d'imagerie à rayons X selon un mode de réalisation.

La figure 1A est une vue de dessus schématique et partielle d'un exemple de réalisation du substrat de report 100 du dispositif d'imagerie.

Sur la figure 1A, seule une portion du substrat de report 100, correspondant à deux pixels adjacents d'une même ligne du dispositif d'imagerie, a été représentée.

Les figures 1B à 1I sont des vues en coupe du dispositif à différents stades de fabrication, selon la ligne de coupe A-A de la figure 1A.

Le substrat de report 100 comprend par exemple une plaque ou feuille de support 101 en un matériau isolant, par exemple en verre ou en plastique. A titre de variante, la plaque ou feuille de support 101 comprend un support conducteur, par exemple métallique, recouvert par une couche d'un matériau isolant. Le substrat de report comprend en outre des éléments de connexion électrique, et en particulier des pistes conductrices et des plots conducteurs, formés sur la face supérieure de la plaque de support 101. Ces éléments de connexion électrique sont par exemple formés par dépôt pleine plaque et gravure d'une succession de niveaux conducteurs et isolants sur la face supérieure de la plaque de support 101. A titre de variante, les éléments de connexion électrique sont formés par impression (ou autre méthode de dépôt localisé) d'une succession de niveaux conducteurs et isolants sur la face supérieure de la plaque de support 101.

Dans l'exemple représenté, le substrat de report 100 comprend deux niveaux métalliques conducteurs M1 et M2 séparés par un niveau isolant I (non représenté sur la figure 1A), et des vias métalliques V (non visibles sur la figure 1B) connectant les deux niveaux métalliques M1 et M2 à travers le niveau isolant I. Dans cet exemple, le substrat de report 100 comprend en outre des plots métalliques de connexion formés sur le niveau métallique supérieur M2, destinés à être connectés à des plots de connexion correspondants des puces élémentaires des pixels du dispositif.

Des circuits actifs de commande du dispositif d'affichage, adaptés à alimenter et commander les puces élémentaires du dispositif par l'intermédiaire des éléments de connexion électrique du substrat de report, sont par exemple connectés aux éléments de connexion électrique du substrat de report à la périphérie du substrat de report 100.

A titre d'exemple, la fabrication du substrat de report 100 comprend les trois étapes successives de dépôt et gravure suivantes.

Lors d'une première étape, une couche conductrice, par exemple métallique, par exemple en titane, en cuivre ou en aluminium, est déposée sur la face supérieure du substrat 101 puis gravée pour former le niveau M1. Dans cet exemple, le niveau M1 comprend une pluralité de pistes conductrices sensiblement parallèles à la direction des colonnes de la matrice de pixels du dispositif d'imagerie (direction verticale dans l'orientation de la figure 1A). Plus particulièrement, dans cet exemple, on forme, dans le niveau M1, pour chaque colonne du dispositif d'imagerie, une piste conductrice C1 s'étendant sur sensiblement toute la longueur des colonnes du dispositif. Chaque piste C1 est destinée à véhiculer un signal VX représentatif de la quantité de charges photogénérées dans les photodiodes des pixels de la colonne correspondante, et donc de l'intensité lumineuse reçue par les photodiodes des pixels de la colonne correspondante.

Lors d'une deuxième étape, on recouvre le niveau M1 par une couche d'un matériau isolant, par exemple de l'oxyde de silicium ou du nitrure de silicium, pour former le niveau isolant I. Des ouvertures localisées sont ensuite gravées dans la couche isolante I aux emplacements des vias V, pour permettre d'établir des connexions électriques entre le niveau M1 et le niveau M2. Les ouvertures dans la couche isolante I sont par exemple formées par gravure humide, par exemple de type BHF (de l'anglais "Buffered Hydrofluoric Acid" - acide fluorhydrique tamponné), ou par gravure plasma.

Lors d'une troisième étape, une couche conductrice, par exemple métallique est déposée sur la face supérieure du niveau isolant I puis gravée pour former le niveau M2. La couche métallique du niveau M2 est de préférence réfléchissante. A titre d'exemple, la couche métallique du niveau M2 est en aluminium. Dans cet exemple, le niveau M2 comprend une pluralité de pistes conductrices sensiblement parallèles à la direction des lignes de la matrice de pixels du dispositif d'imagerie (direction horizontale dans l'orientation de la figure 1A). Plus particulièrement, dans cet exemple, on forme dans le niveau M2, pour chaque ligne du dispositif d'imagerie, une piste conductrice L1 s'étendant sur sensiblement toute la longueur des lignes du dispositif. Chaque piste L1 est destinée à véhiculer un signal SELECT de sélection des photodiodes des pixels de la ligne correspondante.

Dans cet exemple, on forme en outre dans le niveau M2, pour chaque pixel du dispositif, une région métallique E1, définissant une électrode inférieure de la photodiode du pixel.

Après la troisième étape de dépôt, on vient former, pour chaque pixel, sur des zones conductrices du niveau métallique M2, trois plots métalliques P1, P2, P3 destinés à recevoir respectivement trois plots de connexion distincts de la puce élémentaire du pixel. Les plots P1, P2, P3 sont connectés respectivement à la piste conductrice L1 de la ligne de pixels correspondante, à la piste conductrice C1 de la colonne de pixels correspondante, et à l'électrode inférieure E1 de la photodiode du pixel correspondant.

En pratique, les plots métalliques P1, P2, P3 peuvent être formés immédiatement après la formation du niveau M2, ou postérieurement. Dans l'exemple représenté sur les vues en coupe des figures 1B à 1I, les plots métalliques P1, P2, P3 sont formés après la réalisation des photodiodes des pixels (étape de la figure 1E).

La figure 1C illustre la structure obtenue à l'issue d'une étape de formation, dans chaque pixel, d'un empilement actif de diode photosensible 103 sur l'électrode inférieure E1 de la photodiode du pixel.

L'empilement 103 est par exemple un empilement de diode PIN. A titre d'exemple, l'empilement 103 est un empilement à base d'un matériau semiconducteur inorganique pouvant être déposé en film mince sur une surface relativement importante, par exemple du silicium amorphe, ou de l'oxyde d'indium-gallium-zinc (IGZO). A titre de variante, l'empilement 103 est un empilement de photodiode organique comprenant par exemple une couche semiconductrice active organique prise en sandwich entre deux couches de transport de charges (non détaillées sur la figure).

A titre d'exemple, non limitatif, le pas inter-pixels du dispositif dans la direction des lignes et dans la direction des colonnes est compris entre 50 et 500 µm, par exemple entre 100 à 200 µm, par exemple de l'ordre de 150 µm. De préférence, la surface (en vue de dessus) occupée par l'électrode inférieure E1 est supérieure à 50%, de préférence supérieure à 70% de la surface du pixel. L'empilement actif de diode photosensible 103 recouvre sensiblement toute la surface de l'électrode E1.

L'empilement actif 103 est par exemple d'abord déposé de façon continue sur toute la surface du substrat de report, puis retiré de façon localisée, par exemple par photolithographie et gravure, de façon à conserver uniquement des pavés disjoints de l'empilement, localisés en vis-à-vis des électrodes inférieures E1 des photodiodes des pixels.

La figure 1D illustre la structure obtenue à l'issue d'une étape de formation, dans chaque pixel, d'une électrode supérieure E2 sur l'empilement actif de diode photosensible 103 du pixel. L'électrode E2 est en un matériau conducteur transparent, par exemple un oxyde transparent conducteur, par exemple de l'oxyde d'indium-étain (ITO). Dans chaque pixel, l'empilement formé par l'électrode inférieure E1, l'empilement actif 103 et l'électrode supérieure E2 défini une photodiode PD du pixel.

A titre d'exemple, les électrodes E2 des photodiodes PD des pixels du capteur sont toutes interconnectées. Autrement dit, l'électrode supérieure des photodiodes PD est commune à tous les pixels du capteur. Les électrodes inférieures E1 des différentes photodiodes PD sont en revanche distinctes, de façon à permettre une lecture individuelle des photodiodes PD du dispositif.

Dans l'exemple représenté, l'empilement actif de diode photosensible 103 est pixellisé, c'est-à-dire que chaque pixel comprend un pavé formé par une portion de l'empilement 103 séparée latéralement des portions de l'empilement 103 des autres diodes photosensibles PD par des tranchées d'isolation. A titre de variante (non représentée), par exemple dans le cas où l'empilement actif 103 est un empilement actif de diode photosensible organique, l'empilement 103 forme une grille s'étendant de façon continue sur toute la matrice de pixels, la pixellisation étant réalisée uniquement au niveau des électrodes inférieures E1 des pixels.

La figure 1E illustre la structure obtenue à l'issue d'une étape de formation, dans chaque pixel, des plots métalliques de connexion P1, P2, P3 destinés à être fixés et connectés électriquement à des plots métalliques de connexion correspondants de la puce élémentaire du pixel. Dans cet exemple, le plot P1 est formé sur une portion de piste conductrice du niveau M2 connectée à la piste conductrice de colonne C1 du pixel, le plot P2 est formé sur une portion de piste conductrice du niveau M2 connectée, par l'intermédiaire d'un via V, à la piste conductrice de ligne L1 du pixel, et le plot P3 est formé sur une portion de piste conductrice du niveau M2 connectée à l'électrode inférieure E1 de la photodiode PD du pixel.

Dans l'exemple représenté, la face supérieure des plots P1, P2, P3 est située à un niveau plus élevé que la face supérieure de l'électrode supérieure E2 des photodiodes PD des pixels. Autrement dit, le plan de la face supérieure des plots P1, P2, P3 est situé au-dessus du plan de la face supérieure des électrodes E2 des pixels.

Les figures 1F et 1G illustrent une étape de report, dans chaque pixel, d'une puce élémentaire 153 de contrôle et de lecture fixée et connectée électriquement aux plots métalliques de connexion P1, P2, P3 du pixel.

Dans cet exemple, les puces élémentaires 153 sont transférées collectivement depuis un substrat de support temporaire 140 vers le substrat de report 100.

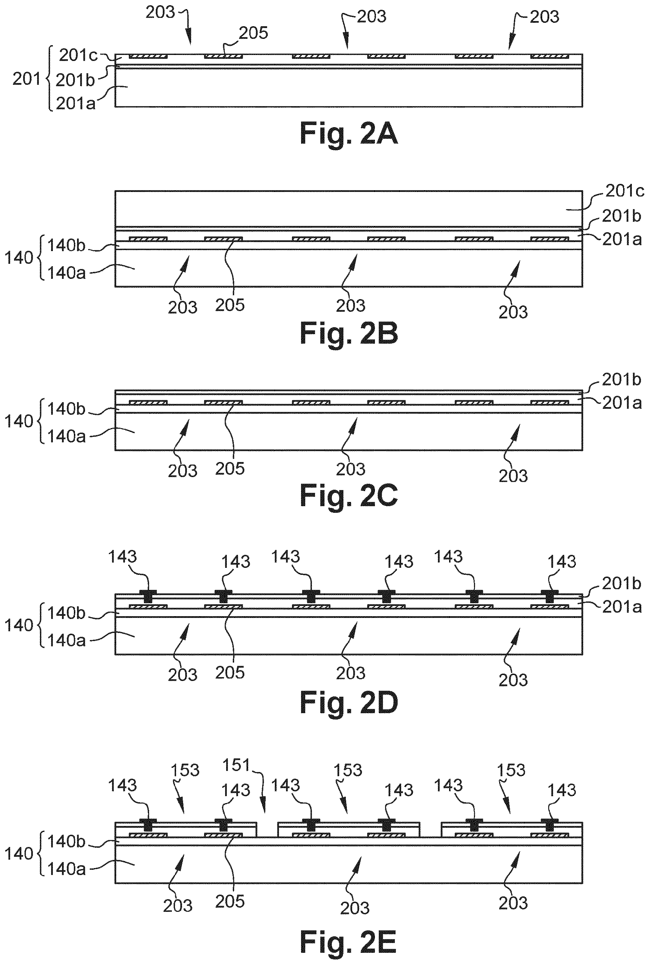

Les puces élémentaires 153 sont initialement fixées à une face du substrat de support temporaire 140 (face inférieure dans l'orientation des figures). La structure comportant le substrat de support temporaire 140 et les puces élémentaires 153 est par exemple réalisée par un procédé du type décrit ci-après en relation avec les figures 2A à 2E.

Chaque puce élémentaire comprend au moins un et de préférence plusieurs transistors MOS, formés dans et sur un substrat semiconducteur, par exemple un substrat en silicium monocristallin. Les puces élémentaires 153 sont par exemple réalisées en technologie CMOS. Chaque puce élémentaire est adaptée à fournir, sur la piste conductrice de colonne C1 du pixel correspondant (via la borne P2), un signal, par exemple une tension, représentatif d'une intensité lumineuse reçue par la photodiode PD du pixel. Les puces 153 peuvent être sélectionnées ligne par ligne, par l'intermédiaire du signal SELECT appliqué sur la piste conductrice L1 correspondante, de façon à lire les photodiodes PD ligne par ligne lors d'une phase d'acquisition d'une image.

Les puces élémentaires 153 sont rapportées collectivement en vis-à-vis de la face de connexion du substrat de report 100, à savoir sa face supérieure dans l'orientation des figures, en utilisant le substrat de support temporaire 140 comme poignée (figure 1F).

Des plots de connexion 143 de puces élémentaires 153, situés du côté de la face inférieure desdites puces, sont alors mis en contact avec les plots de connexion correspondants P1, P2, P3 du substrat de report 100, et fixés auxdits plots de connexion P1, P2, P3. La fixation des plots de connexion 143 des puces élémentaires 153 aux plots de connexion du substrat de report est par exemple réalisée par collage direct, par thermocompression, par brasure, au moyen de microstructure métalliques (par exemple des micro-piliers) préalablement formées sur les plots 143, ou par toute autre méthode de fixation et de connexion adaptée, par exemple par connexion au moyen d'un film conducteur de type AFC (de l'anglais "Anisotropic Conducting Film" - film conducteur anisotrope).

Une fois fixées, par leurs plots de connexion 143, au substrat de report 100, les puces élémentaires 153 sont détachées du substrat de support temporaire 140, et ce dernier est retiré (figure 1G), libérant l'accès à la face d'éclairement des photodiodes PD.

Le pas des puces élémentaires 153 sur le substrat de report 100 peut être supérieur au pas des puces élémentaires 153 sur le substrat de support temporaire 140. De préférence, le pas des puces élémentaires 153 sur le substrat de report 100 est un multiple du pas des puces élémentaires 153 sur le substrat de support temporaire 140. Dans ce cas, seule une partie des puces 153 est prélevée sur le substrat de support 140 à chaque transfert, comme illustré sur les figures 1F et 1G. Les autres puces 153 restent solidaires du substrat de support temporaire 140 et peuvent être utilisées lors d'une autre étape de report collectif pour peupler une autre partie du substrat de report 100 ou un autre substrat de report.

La figure 1G illustre une étape de dépôt d'une couche de planarisation 170 sur la structure obtenue à l'issue des étapes des figures 1A à 1G. Le matériau de la couche 170 est un matériau diélectrique transparent, par exemple un matériau polymère. Le matériau de la couche 170 s'étend depuis la face supérieure du substrat de support, jusqu'à une hauteur supérieure à celle de la face supérieure des puces élémentaires 153. Ainsi, le matériau de la couche 170 recouvre entièrement le substrat de support, les photodiodes PD, et les puces élémentaires 153. La face supérieure de la couche 170 est sensiblement plane et s'étend de façon continue sur toute la surface de la matrice de pixels.

La figure 1I illustre la structure obtenue à l'issue d'une étape de dépôt d'un scintillateur 180 sur la face supérieure de la couche de planarisation 170. Le scintillateur 180 comprend une couche d'un matériau de scintillation, par exemple de l'iodure de césium (CsI) sous forme cristalline, de l'oxyde de gadolinium (GadOx), ou tout autre matériau de scintillation adapté, c'est-à-dire un matériau émettant de la lumière à la suite d'un dépôt d'énergie par interaction avec des rayons X, s'étendant de façon continue sur toute la surface du substrat de report 100.

On notera que les figures ne sont par représentées à l'échelle. A titre d'exemple, la couche de scintillation peut avoir une épaisseur comprise entre 200 µm et 1 mm selon les applications visées, par exemple de l'ordre de 600 µm. L'épaisseur des photodiodes PD est par exemple comprise entre 1 et 10 µm, par exemple entre 1 et 2 µm pour des photodiodes à base de silicium amorphe, ou d'oxyde d'indium-gallium-zinc (IGZO), et entre 1 et 5 µm pour des photodiodes organiques. L'épaisseur des puces élémentaires 153 est par exemple comprise entre 100 µm et 500 µm. Les dimensions latérales des puces élémentaires 153 sont par exemple comprises entre 5 et 150 µm, par exemple entre 10 et 60 µm.

Le scintillateur 180 est par exemple formé séparément sur un substrat de croissance, puis reporté sur la face supérieure de la couche de passivation 170. A titre de variante, le scintillateur 180 est formé directement sur la face supérieure de la couche de passivation 170.

Les figures 2A, 2B, 2C, 2D et 2E sont des vues en coupe illustrant des étapes successives d'un exemple d'un procédé de fabrication des puces élémentaires 153 d'un dispositif d'imagerie à rayons X du type décrit en relation avec les figures 1A à 1I.

La figure 2A représente de façon schématique une structure de commande comprenant un premier substrat 201 dans et sur lequel ont été formés une pluralité de circuits intégrés élémentaires de contrôle 203, par exemple identiques ou similaires, correspondant respectivement aux circuits intégrés de contrôle des futures puces élémentaires 153 des pixels du dispositif.

Dans l'exemple représenté, le substrat 201 est un substrat de type SOI (de l'anglais "Semiconductor On Insulator" - semiconducteur sur isolant), comportant un substrat semiconducteur de support 201a, par exemple en silicium, une couche isolante 201b, par exemple en oxyde de silicium, disposée sur et en contact avec la face supérieure du substrat de support 201a, et une couche semiconductrice supérieure 201c, par exemple en silicium monocristallin, disposée sur et en contact avec la face supérieure de la couche isolante 201b.

Dans cet exemple, les circuits élémentaires de contrôle 203 sont formés dans et sur la couche semiconductrice supérieure 201c du substrat 201. Chaque circuit élémentaire de contrôle 203 comprend par exemple un ou plusieurs transistors MOS (non détaillés sur les figures). Les circuits élémentaires de contrôle 203 sont par exemple réalisés en technologie CMOS (de l'anglais "Complementary Metal Oxyde Semiconductor" - métal oxyde semiconducteur complémentaire). Chaque circuit élémentaire de contrôle 203 peut comprendre un circuit de lecture d'une photodiode PD du dispositif d'imagerie.

La figure 2B illustre la structure obtenue à l'issue d'une étape de report et de fixation de la structure de la figure 2A sur le substrat de support temporaire 140.

Sur la figure 2B, l'orientation de la structure de la figure 2A est inversée par rapport à la figure 2A.

Dans cet exemple, le substrat de support temporaire 140 comprend une première couche 140a d'un matériau de support, par exemple du verre ou du silicium, d'épaisseur comprise, par exemple, entre 200 et 700 µm, et une deuxième couche 140b, plus mince, en un matériau adhésif d'adhérence relativement faible pour permettre le détachement sélectif des puces élémentaires lors de l'étape de transfert collectif des figures 1F et 1G, par exemple un matériau polymère. Dans cet exemple, la couche 140b est disposée sur et en contact avec la face supérieure de la couche 140a. La structure comprenant les circuits de contrôle 203 est fixée à la face supérieure de la couche 140b par sa face inférieure, à savoir sa face opposée au support 201a (correspondant à sa face supérieure dans l'orientation de la figure 2A).

La figure 2C illustre la structure obtenue après une étape de retrait du support 101c de la structure SOI de départ, par exemple par meulage et/ou gravure chimique, de façon à libérer l'accès à la face supérieure de la couche isolante 201b de la structure SOI.

On notera que les modes de réalisation décrits ne se limitent pas à l'exemple décrit ci-dessus dans lequel le substrat 201 est un substrat de type SOI. A titre de variante, le substrat 201 peut être un substrat semiconducteur massif, par exemple en silicium. Dans ce cas, à l'étape de la figure 2C, le substrat 201 peut être aminci par sa face arrière (face supérieure dans l'orientation de la figure 2C), par exemple par meulage. Une couche isolante de passivation, par exemple en oxyde de silicium, peut ensuite être déposée sur la face supérieure du substrat aminci, remplaçant la couche 201b du substrat SOI.

La figure 2D illustre la structure obtenue à l'issue d'étapes de formation d'ouvertures de reprise de contact dans les couches 201b et 201c, et de formation de métallisations de reprise de contact 143 dans et sur lesdites ouvertures. Les métallisations 143 permettent de reprendre des contacts électriques sur des niveaux métalliques (non détaillés sur les figures) de l'empilement d'interconnexion disposé du côté de la face inférieure de la couche semiconductrice 201c. Les métallisations 143 sont par exemple connectées électriquement à des transistors du circuit de contrôle, ces transistors étant eux-mêmes connectés ou reliés électriquement à des métallisations de connexion 205 des circuits 203.

Les métallisations 143 forment des bornes de connexion des futures puces élémentaires des pixels du dispositif, destinées à être connectées à des bornes de connexion correspondantes du substrat de report 100 du dispositif.

La figure 2E illustre la structure obtenue à l'issue d'une étape de singularisation des puces élémentaires de pixel du dispositif. Pour cela, des tranchées 151 s'étendant verticalement à travers les couches 201b et 201c sont formées à partir de la face supérieure de la structure, selon des lignes de découpe. Dans cet exemple, les tranchées débouchent sur la face supérieure du substrat de support temporaire 140. En vue de dessus, les tranchées 151 forment une grille continue délimitant latéralement une pluralité de puces élémentaires de pixel 153, par exemple identiques ou similaires, comportant chacune un circuit de contrôle élémentaire 203. Les tranchées 151 sont par exemple réalisées par gravure plasma.

Les puces élémentaires 153 sont destinées à être reportées sur le substrat de report 100 du dispositif d'imagerie à rayons X, comme cela a été décrit ci-dessus en relation avec les figures 1A à 1I.

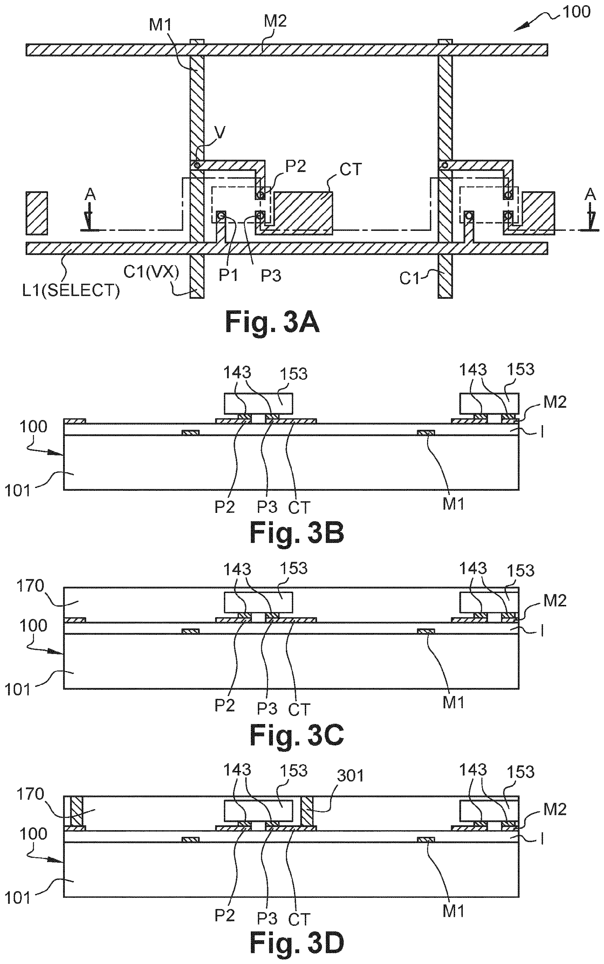

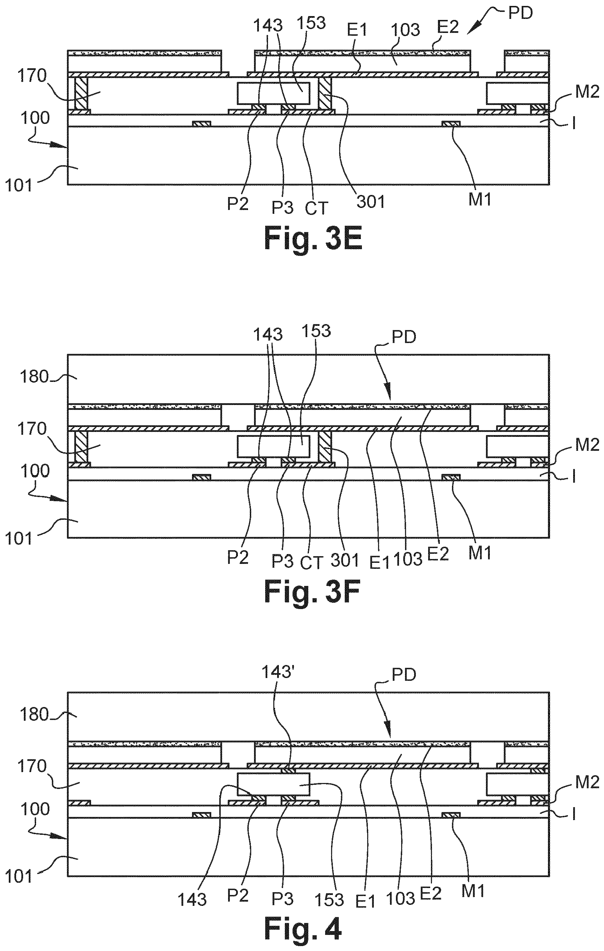

Les figures 3A, 3B, 3C, 3D, 3E et 3F sont des vues de dessus et en coupe illustrant des étapes d'un autre exemple d'un procédé de fabrication d'un dispositif d'imagerie à rayons X selon un mode de réalisation.

Le procédé des figures 3A à 3F diffère du procédé des figures 1A à 1I principalement en ce que, dans le procédé des figures 3A à 3F, les puces élémentaires 153 des pixels sont reportées sur le substrat de report 100 avant la formation des photodiodes PD, et non pas après comme dans l'exemple des figures 1A à 1I.

La figure 3A est une vue de dessus schématique et partielle d'un exemple de réalisation du substrat de report 100 du dispositif d'imagerie.

Les figures 3B à 3F sont des vues en coupe selon la ligne A-A de la figure 3A.

Comme dans l'exemple des figures 1A à 1I, le substrat de report 100 comprend deux niveaux métalliques conducteurs M1 et M2 séparés par un niveau isolant I, et des vias métalliques V connectant les deux niveaux métalliques M1 et M2 à travers le niveau isolant I. Dans cet exemple, le substrat de report 101 comprend en outre des plages métalliques de connexion formées sur le niveau métallique supérieur M2, destinées à être connectées à des plages de connexion correspondantes des puces élémentaires des pixels du dispositif.

A titre d'exemple, de façon similaire à ce qui a été décrit en relation avec les figures 1A à 1I, le niveau M1 comprend, pour chaque colonne de pixels du dispositif d'imagerie, une piste conductrice C1 s'étendant sur sensiblement toute la longueur des colonnes du dispositif, destinée à véhiculer un signal VX représentatif de l'intensité lumineuse reçue par les photodiodes des pixels de la colonne correspondante, et le niveau M2 comprend, pour chaque ligne de pixels du dispositif d'imagerie, une piste conductrice L1 s'étendant sur sensiblement toute la longueur des lignes du dispositif, destinée à véhiculer un signal SELECT de sélection des photodiodes des pixels de la ligne correspondante.

Dans cet exemple, est en outre formée dans le niveau M2, pour chaque pixel du dispositif, une région métallique CT, définissant une région de reprise de contact destinée à être connectée électriquement à une électrode inférieure de la photodiode du pixel. A la différence de l'exemple des figures 1A à 1I, la région CT ne constitue pas directement l'électrode inférieure de la photodiode du pixel. En particulier, la région CT peut présenter une surface inférieure à celle de l'électrode inférieure E1 de la photodiode du pixel.

Après la formation du niveau M2, on vient former, pour chaque pixel, sur des zones conductrices du niveau métallique M2, trois plots métalliques P1, P2, P3 destinés à recevoir respectivement trois plots de connexion distincts de la puce élémentaire du pixel. Les plots P1, P2, P3 sont connectés respectivement à la piste conductrice L1 de la ligne de pixels correspondante, à la piste conductrice C1 de la colonne de pixels correspondante, et à la région CT de reprise de contact sur l'électrode inférieur de la photodiode du pixel correspondant.

A titre de variante, les plots de connexion P1, P2, P3 peuvent être constitués directement par des portions du niveau M2.

Dans cet exemple, le plot P1 est connecté à la piste conductrice de colonne C1 du pixel par l'intermédiaire d'une portion de piste conductrice du niveau M2, le plot P2 est connecté à la piste conductrice de ligne L1 du pixel par l'intermédiaire d'une portion de piste conductrice du niveau M2 et d'un via V, et le plot P3 est connecté à la région CT par une portion de piste conductrice du niveau M2.

La figure 3B illustre la structure obtenue à l'issue d'une étape de report, dans chaque pixel, d'une puce élémentaire 153 de contrôle et de lecture fixée et connectée électriquement aux plots métalliques de connexion P1, P2, P3 du pixel.

Les puces 153 sont par exemple transférées collectivement depuis un substrat de support temporaire, de façon similaire à ce qui a été décrit ci-dessus en relation avec les figures 1F et 1G.

La figure 3C illustre une étape de dépôt d'une couche de planarisation 170 sur la structure obtenue à l'issue des étapes des figures 3A et 3B. Le matériau de la couche 170 est un matériau diélectrique, transparent ou non, par exemple un matériau polymère. Le matériau de la couche 170 s'étend depuis la face supérieure du substrat de support, jusqu'à une hauteur supérieure à celle de la face supérieure des puces élémentaires 153. Ainsi, le matériau de la couche 170 recouvre entièrement le substrat de support et les puces élémentaires 153. La face supérieure de la couche 170 est sensiblement plane et s'étend de façon continue sur toute la surface du substrat de report 100.

La figure 3C illustre en outre une étape de formation, dans chaque pixel, d'un via conducteur 301 s'étendant verticalement à travers la couche 170. Le via 301 est en contact, par sa face inférieure, avec la face supérieure de la région de contact CT. La face supérieure du via 301 affleure au niveau de la face supérieure de la couche 170.

La figure 3E illustre la structure obtenue à l'issue des étapes successives de :
- formation, dans chaque pixel, de l'électrode inférieure E1 de la photodiode du pixel ;
- formation, dans chaque pixel, d'un empilement actif de photodiode 103 sur et en contact avec la face supérieure de l'électrode E1 ; et
- formation, dans chaque pixel, de l'électrode supérieure E2 de la photodiode du pixel.

L'électrode E1, l'empilement actif 103, et l'électrode supérieure E2 sont par exemple identiques ou similaires à ce qui a été décrit ci-dessus en relation avec les figures 1B, 1C et 1D.

Dans chaque pixel, l'électrode inférieure E1 est en contact, par sa face inférieure, avec la face supérieure du via 301 du pixel. Ainsi, l'électrode inférieure E1 est connectée électriquement à la région de reprise de contact CT du pixel (et donc la puce élémentaire 153 du pixel) par l'intermédiaire du via 301.

Dans chaque pixel, l'empilement formé par l'électrode inférieure E1, l'empilement actif 103 et l'électrode supérieure E2 définit une photodiode PD du pixel.

A titre d'exemple, les électrodes E2 des photodiodes PD des pixels du capteur sont toutes interconnectées. Autrement dit, l'électrode supérieure des photodiodes PD est commune à tous les pixels du capteur. Les électrodes inférieures E1 des différentes photodiodes PD sont en revanche distinctes, de façon à permettre une lecture individuelle des photodiodes PD du dispositif.

De préférence, la photodiode PD, et plus particulièrement l'empilement actif 103 de la photodiode PD, s'étendent au-dessus de la puce élémentaire 153 du pixel.

Ceci permet, à pas inter-pixel équivalent, d'augmenter la surface de photo-détection du pixel par rapport à l'exemple des figures 1A à 1I.

Dans l'exemple représenté, l'empilement actif de diode photosensible 103 est pixellisé, c'est-à-dire que chaque pixel comprend un pavé formé par une portion de l'empilement 103 séparée latéralement des portions de l'empilement 103 des autres diodes photosensibles PD par des tranchées d'isolation. A titre de variante (non représentée), par exemple dans le cas où l'empilement actif 103 est un empilement actif de diode photosensible organique, l'empilement 103 s'étend de façon continue sur toute la matrice de pixels, la pixellisation étant réalisée uniquement au niveau des électrodes inférieures E1 des pixels.

La figure 3F illustre la structure obtenue à l'issue d'une étape de dépôt d'un scintillateur 180, par exemple identique ou similaire à celui de la figure 1I, au-dessus des photodiodes PD.

Dans cet exemple, le scintillateur 180 est déposé directement au-dessus des photodiodes PD, sans couche de planarisation intermédiaire.

A titre de variante, une couche de planarisation transparente peut être déposée au-dessus des photodiodes PD avant le dépôt du scintillateur 180.

La figure 4 est une vue en coupe illustrant une variante du procédé des figures 3A, 3B, 3C, 3D, 3E et 3F.

Dans cette variante, dans chaque pixel, la connexion électrique entre la puce élémentaire 153 et l'électrode inférieure E1 de la photodiode PD du pixel est réalisée par l'intermédiaire d'une borne métallique de connexion 143' de la puce élémentaire, située en face supérieure de la puce élémentaire. La borne de connexion 143' affleure au niveau de la face supérieure de la couche de planarisation 170. La borne de connexion 143' est en contact, par sa face supérieure, avec la face inférieure de l'électrode E1. Ainsi, le via conducteur 301 et la région métallique de contact CT de l'exemple des figures 3A à 3F peuvent être omis.

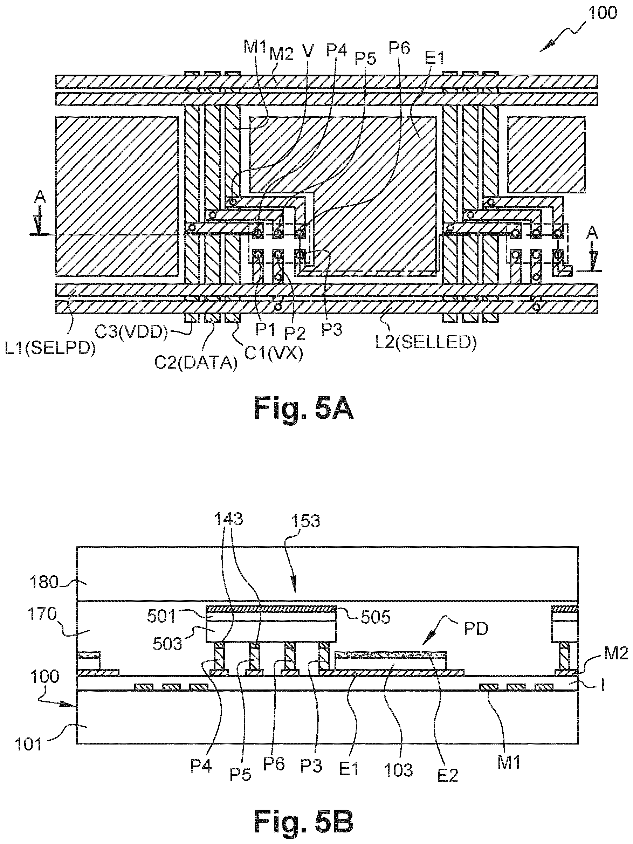

Les figures 5A et 5B sont des vues de dessus et en coupe illustrant des étapes d'un autre exemple d'un procédé de fabrication d'un dispositif d'imagerie à rayons X selon un mode de réalisation.

La figure 5A est une vue de dessus schématique et partielle d'un exemple de réalisation du substrat de report 100 du dispositif d'imagerie.

La figure 5B est une vue en coupe du dispositif selon la ligne de coupe A-A de la figure 5A.

Le procédé des figures 5A et 5B présente des étapes identiques ou similaires aux étapes du procédé des figures 1A à 1I. Ces étapes ne seront pas détaillées à nouveau ci-après et seules les différences par rapport au procédé des figures 1A à 1I seront mises en exergue.

L'exemple des figure 5A et 5B diffère de l'exemple des figures 1A à 1I principalement en ce que, dans l'exemple des figures 5A et 5B, dans chaque pixel, chaque puce élémentaire 153 rapportée sur le substrat de report comprend non seulement un circuit intégré de contrôle et de lecture de la photodiode PD du pixel, mais aussi une diode électroluminescente (LED) inorganique, et un circuit intégré de contrôle de la LED.

L'intégration d'une LED dans la puce élémentaire 153 permet avantageusement de mettre en oeuvre, entre deux phases d'acquisition d'une image, une étape de réinitialisation des photodiodes PD par application d'un flash lumineux sur les photodiodes PD.

Les puces élémentaires 153 sont des puces de pixel monolithiques, par exemple du type décrit dans les demandes de brevet WO2017089676, EP3401958 et WO2018185433 précédemment déposées. Chaque puce comporte une LED 501 et un circuit élémentaire de contrôle 503 accolé et connecté électriquement à la LED. Le circuit de contrôle 503 est par exemple réalisé en technologie CMOS. Le circuit 503 comprend par exemple un circuit de contrôle et de lecture de la photodiode PD du pixel, et un circuit de commande de la LED.

Dans cet exemple, la LED 501 recouvre la face supérieure du circuit élémentaire de contrôle 503. Le circuit 503 comporte des bornes de connexion 143 du côté de sa face inférieure.

Dans l'exemple représenté, la LED 501 est recouverte, du côté de sa face supérieure, par une couche opaque ou réfléchissante 505, par exemple en métal. La couche 505 permet d'orienter la lumière émise par la LED en direction de la photodiode PD du pixel. La couche 505 forme par exemple l'électrode supérieure de la LED 501.

Dans l'exemple des figures 5A et 5B, la couche de planarisation 170 (figure 5B) est en un matériau transparent.

Pour permettre de lire les photodiodes PD des pixels et de commander les LED 501 des pixels en émission, le substrat de report 100 de l'exemple des figures 5A et 5B comprend un nombre de piste conductrices et de métallisations de connexion supérieur à celui de l'exemple des figures 1A à 1I. De plus, les puces élémentaires 153 comportent un nombre de bornes de connexion supérieur à ce qui a été décrit précédemment.

Dans l'exemple représenté, chaque puce élémentaire comprend six bornes de connexion destinées à être connectées respectivement à six plots métalliques de connexion P1, P2, P3, P4, P5, P6 du substrat de report 100.

A titre d'exemple, le niveau M1 comprend, pour chaque colonne de pixels du dispositif d'imagerie, trois pistes conductrices de colonne C1, C2, C3 destinées à véhiculer respectivement un signal VX représentatif de l'intensité lumineuse reçue par les photodiodes des pixels de la colonne correspondante, un signal DATA de commande des LED des pixels de la colonne correspondante, et un signal VDD d'alimentation des LED des pixels de la colonne correspondante. Dans cet exemple, le niveau M2 comprend, pour chaque ligne de pixels du dispositif d'imagerie, deux pistes conductrices de ligne L1 et L2 destinées à véhiculer respectivement un signal SELPD de sélection des photodiodes des pixels de la ligne correspondante, et un signal SELLED de sélection des LED des pixels de la ligne correspondante.

Les plots métalliques P1, P2, P3, P4, P5, P6 sont formés sur des zones conductrices du niveau métallique M2 et sont destinés à recevoir respectivement six plots de connexion distincts de la puce élémentaire du pixel. Les plots P1, P2, P3, P4, P5, P6 sont connectés respectivement à la piste conductrice L1 de la ligne de pixels correspondante, à la piste conductrice L2 de la ligne de pixels correspondante, à l'électrode E1 de la photodiode PD du pixel correspondant, à la piste conductrice C3 de la colonne de pixels correspondante, à la piste conductrice C4 de la colonne de pixels correspondante, et à la piste conductrice C5 de la colonne de pixels correspondante.

Dans l'exemple des figures 5A et 5B, de façon similaire à ce qui a été décrit ci-avant en relation avec les figures 1A à 1I, les puces élémentaires 153 sont fixées et connectées électriquement au substrat de report 100 avant la formation des photodiodes PD des pixels.

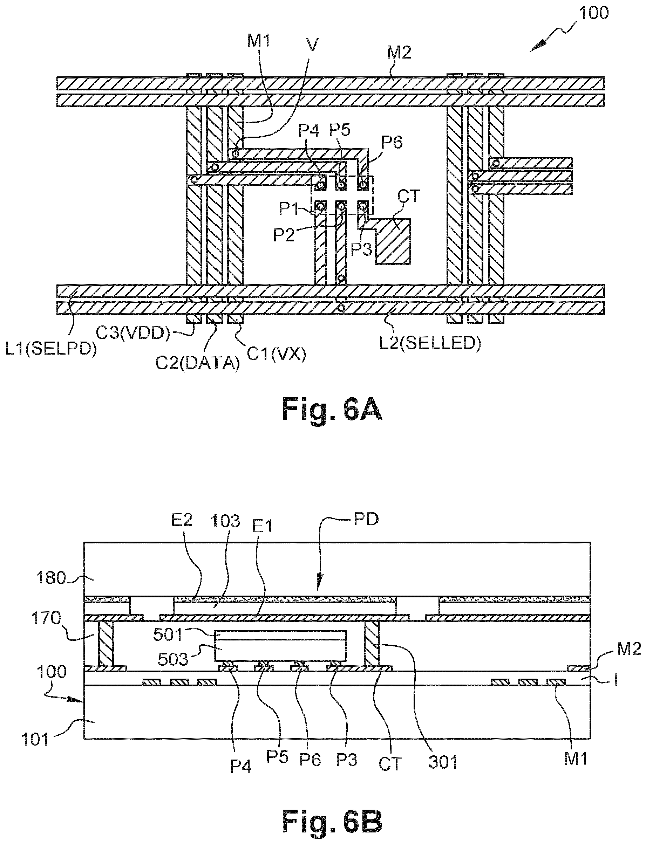

Les figures 6A et 6B sont des vues de dessus et en coupe illustrant des étapes d'une variante de réalisation du procédé des figures 5A et 5B, dans laquelle les puces élémentaires sont fixées et connectées électriquement au substrat du report après la formation des photodiodes PD, de façon similaire à ce qui a été décrit ci-avant en relation avec les figures 3A à 3F.

On notera que dans cette variante, la couche réfléchissante 505 de la figure 5B peut être omise ou remplacée par une couche transparente, par exemple une couche conductrice transparente, par exemple en ITO, formant l'électrode supérieure de la LED 501. En effet, dans chaque pixel, la LED élémentaire 501 du pixel est située directement sous la photodiode PD du pixel.

Dans l'exemple des figures 6A et 6B, la couche de planarisation 170 (figure 6B) est en un matériau transparent.

On notera que la variante des figures 6A et 6B peut être combinée à la variante de la figure 4, auquel cas les éléments de connexion CT et 301 de la figure 6B peuvent être omis.

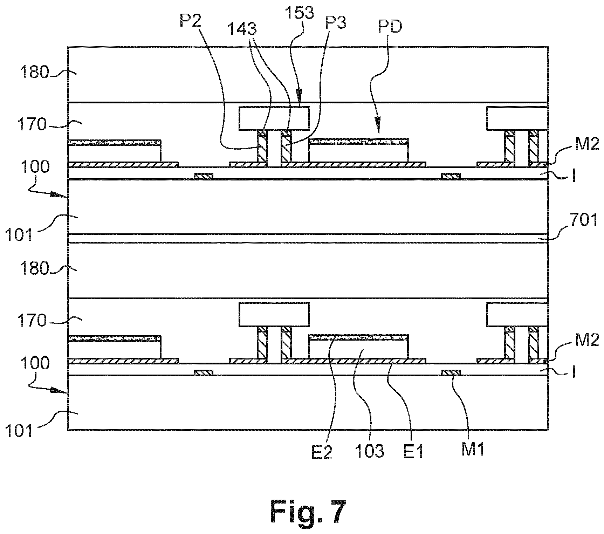

La figure 7 est une vue en coupe illustrant une autre variante de réalisation d'un dispositif d'imagerie à rayons X selon un mode de réalisation. Dans cet exemple, le dispositif comprend deux dispositifs du type décrit en relation avec la figure 1I, superposés.

Le dispositif de la figure 7 permet de faire de l'imagerie à rayons X biénergie, aussi appelée imagerie à rayons X couleur, c'est-à-dire d'imager respectivement un premier niveau d'énergie, appelé niveau de basse énergie (BE), au moyen du dispositif d'imagerie supérieur, et un deuxième niveau d'énergie, appelé niveau de haute énergie (HE), au moyen du dispositif d'imagerie inférieur. A titre d'exemple, le dispositif d'imagerie supérieur est adapté à détecter des rayonnements de niveau d'énergie compris entre 1 keV et 140 keV, par exemple entre 40 keV et 80 keV, par exemple de l'ordre de 60 keV en moyenne, et le dispositif d'imagerie inférieur est adapté à détecter des rayonnements de niveau d'énergie compris entre 60 keV et 140 keV, par exemple entre 80 keV et 120 keV, par exemple de l'ordre de 100 keV en moyenne.

Dans l'exemple représenté, une couche d'interface 701 est disposée entre la face inférieure du substrat de support 101 du dispositif supérieur et la face supérieure du scintillateur 180 du dispositif inférieur. L'épaisseur du substrat de support du dispositif supérieur est de préférence relativement faible pour limiter l'absorption des photons de haute énergie. A titre d'exemple, l'épaisseur du substrat de support du dispositif supérieur est inférieure à l'épaisseur du substrat de support du dispositif inférieur.

La couche d'interface 710 peut comprendre une couche de filtrage adaptée à filtrer le rayonnement basse-énergie de façon que seul le rayonnement haute-énergie parvienne sur le dispositif d'imagerie inférieur. La couche de filtrage est par exemple une couche métallique, par exemple continue, par exemple en cuivre ou en aluminium, par exemple d'épaisseur comprise entre 0,1 et 0,4 mm. La couche de filtrage permet d'améliorer la séparation spectrale entre les deux dispositifs d'imagerie.

A titre de variante, la couche d'interface 701 peut être omise.

Le mode de réalisation de la figure 7 peut bien entendu être combiné à toutes les variantes décrites précédemment.

Divers modes de réalisation et variantes ont été décrits. La personne du métier comprendra que certaines caractéristiques de ces divers modes de réalisation et variantes pourraient être combinées, et d'autres variantes apparaîtront à la personne du métier. En particulier, les modes de réalisation décrits ne se limitent pas aux exemples de dimensions et de matériaux mentionnés dans la description

Par ailleurs, on a décrit ci-dessus des exemples de réalisation dans lesquels un scintillateur 180 recouvre une matrice de pixels comprenant chacun une puce élémentaire monolithique et une photodiode. A titre de variante, dans chaque pixel, le détecteur comprend un empilement actif de détection à base d'un matériau scintillateur 180 adapté à convertir directement des photons X en photons lumineux, par exemple un matériau du groupe comprenant l'Iodure de Cesium (CsI :Tl) ou du GADOX (Gd2O2S:Tb), lesquels photons lumineux interagissent ensuite avec la photodiode du pixel pour générer des électrons.

## Revendications

1. Dispositif d'imagerie à rayons X, comportant :
- un substrat de report (100) comportant des éléments de connexion électrique ;
- une matrice de pixels comportant chacun une puce élémentaire monolithique (153) fixée et connectée électriquement à des éléments de connexion électrique du substrat de report (100), et une photodiode (PD) formée sur le substrat de report et connectée électriquement à la puce élémentaire (153) ; et
- un scintillateur (180) revêtant chaque pixel,
dans lequel, dans chaque pixel, la puce élémentaire (153) comprend un circuit intégré de lecture de la photodiode (PD) du pixel.

2. Dispositif selon la revendication 1, dans lequel, dans chaque puce élémentaire (153), le circuit intégré de lecture de la photodiode (PD) du pixel est réalisé en technologie CMOS.

3. Dispositif selon la revendication 1 ou 2, dans lequel, dans chaque pixel, la photodiode (PD) comprend un empilement actif (103) à base d'un matériau semiconducteur inorganique, par exemple du silicium amorphe ou de l'oxyde d'indium-gallium-zinc.

4. Dispositif selon la revendication 1 ou 2, dans lequel, dans chaque pixel, la photodiode (PD) comprend un empilement actif (103) de diode photosensible organique.

5. Dispositif selon l'une quelconque des revendications 1 à 4, dans lequel, dans chaque pixel, la photodiode (PD) comprend une électrode supérieure (E2) en un matériau transparent.

6. Dispositif selon l'une quelconque des revendications 1 à 5, dans lequel, dans chaque pixel, la photodiode (PD) ne recouvre pas la puce élémentaire (153) du pixel.

7. Dispositif selon l'une quelconque des revendications 1 à 5, dans lequel, dans chaque pixel, la photodiode (PD) recouvre la puce élémentaire (153) du pixel.

8. Dispositif selon l'une quelconque des revendications 1 à 7, dans lequel, dans chaque pixel, la puce élémentaire (153) du pixel comprend une LED inorganique (501) et un circuit intégré (503) de contrôle de la LED.

9. Assemblage comportant des premier et deuxième dispositifs d'imagerie à rayons X selon l'une quelconque des revendications 1 à 8, superposés.

10. Assemblage selon la revendication 9, comprenant une couche de filtrage (701) entre les premier et deuxième dispositifs.

11. Procédé de fabrication d'un dispositif d'imagerie à rayons X selon l'une quelconque des revendications 1 à 8, dans lequel les puces élémentaires (153) sont reportées et fixées collectivement au substrat de report (100), au moyen d'un substrat de support temporaire (140).

12. Procédé selon la revendication 9, comprenant la formation des photodiodes (PD) des pixels sur le substrat de report (100) avant l'étape de report collectif des puces élémentaires (153) sur le substrat de report.

13. Procédé selon la revendication 9, comprenant la formation des photodiodes (PD) des pixels sur le substrat de report (100) après l'étape de report collectif des puces élémentaires (153) sur le substrat de report.
